# EUROPÄISCHE PATENTANMELDUNG

(11) **EP 1 496 046 A1**
(43) Veröffentlichungstag der Anmeldung: **12.01.2005**
(21) Anmeldenummer: 04015350.4
(22) Anmeldetag: 30.06.2004
(51) Int. Cl.: C07C 215/16, A61K 7/13

(54) **Neue Kupplerkomponenten**

(30) Priorität: 08.07.2003 DE 10330687
(71) Anmelder: Henkel Kommanditgesellschaft auf Aktien, 40589 Düsseldorf (DE)
(72) Erfinder: Knübel, Georg, 40219 Düsseldorf (DE); Meinigke, Bernd, 51381 Leverkusen (DE); Höffkes, Horst, 40595 Düsseldorf (DE); Nemitz, Ralph, 61363 Jüchen (DE)

(57) **Zusammenfassung**

Gegenstand der vorliegenden Anmeldung sind Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 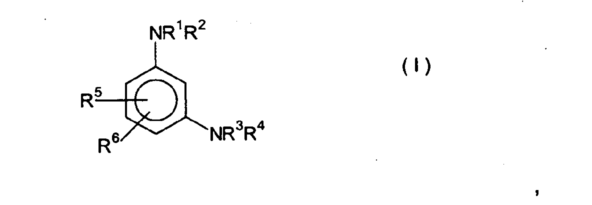 wobei
R¹, R², R³ und R⁴ stehen unabhängig voneinander für ein Wasserstoffatom, eine C₁- bis C₄-Alkylgruppe, eine C₂- bis C₄-Monohydroxyalkylgruppe oder R¹ und R² beziehungsweise R³ und R⁴ bilden gemeinsam mit dem sie tragenden Stickstoffatom einen Pyrrolidin-, einen Piperidin- oder Morpholinring, mit der Maßgabe, dass einer der Substituenten R¹, R², R³ oder R⁴ für eine lineare oder verzweigte, chirale oder achirale C₅- bis C₈-Monohydroxyalkylgruppe (X) steht, und
R⁵ und R⁶ stehen unabhängig voneinander für ein Wasserstoffatom, eine C₁- bis C₂-Alkylgruppe, eine C₁- bis C₄-Monohydroxyalkylgruppe oder eine C₁- bis C₂-Alkoxygruppe. Die erfindungsgemäßen m-Phenylendiaminderivate ermöglichen, insbesondere mit p-Phenylendiamin- und Diaminopyrazolderivaten, gute Lichtechtheiten sowie ein gutes Egalisiervermögen.

## Beschreibung

Die vorliegende Erfindung betrifft Mittel zur Färbung keratinischer Fasern, die spezielle m-Phenylendiaminderivate enthalten, die eine lineare oder verzweigte, chirale oder achirale C₅- bis C₈-Monohydroxyalkylgruppe tragen, ein Verfahren zur Färbung von Haaren mit diesen Mitteln sowie einige dieser m-Phenylendiaminderivate selbst.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluss chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, die auch in toxikologischer und dermatologischer Hinsicht unproblematisch sind.

Aufgabe der vorliegenden Erfindung war es daher, neue Kupplerkomponenten zu entwickeln, die die an Oxidationsfarbstoffvorprodukte gestellten Anforderungen, insbesondere hinsichtlich der toxikologischen und dermatologischen Eigenschaften, erfüllen und Färbungen in einem breiten Farbspektrum mit guten Echtheitseigenschaften ermöglichen.

Überraschenderweise wurde nun gefunden, dass m-Phenylendiaminderivate, die eine lineare oder verzweigte, chirale oder achirale C₅- bis C₈-Monohydroxyalkylgruppe tragen, den an Kupplerkomponenten gestellten Anforderungen in einem hohen Maße genügen.

Die erfindungsgemäßen Kupplerkomponenten ermöglichen, insbesondere mit p-Phenylendiamin- und Diaminopyrazolderivaten, gute Lichtechtheiten sowie ein gutes Egalisiervermögen.

Ein erster Gegenstand der vorliegenden Erfindung sind daher Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 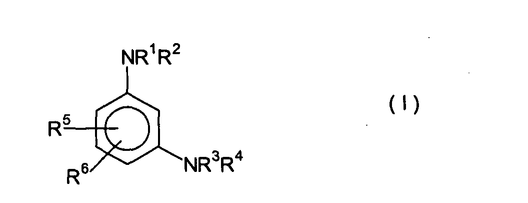 wobei
R¹, R², R³ und R⁴ stehen unabhängig voneinander für ein Wasserstoffatom, eine C₁- bis C₄-Alkylgruppe, eine C₂- bis C₄-Monohydroxyalkylgruppe oder R¹ und R² beziehungsweise R³ und R⁴ bilden gemeinsam mit dem sie tragenden Stickstoffatom einen Pyrrolidin-, einen Piperidin- oder Morpholinring, mit der Maßgabe, dass einer der Substituenten R¹, R², R³ oder R⁴ für eine lineare oder verzweigte, chirale oder achirale C₅- bis C₈-Monohydroxyalkylgruppe (X) steht, und
R⁵ und R⁶ stehen unabhängig voneinander für ein Wasserstoffatom, eine C₁- bis C₂-Alkylgruppe, eine C₁- bis C₄-Monohydroxyalkylgruppe oder eine C₁- bis C₂-Alkoxygruppe.

Unter keratinischen Fasern sind dabei erfindungsgemäß Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Da es sich bei den erfindungsgemäßen m-Phenylendiaminderivaten um AminoVerbindungen handelt, lassen sich aus diesen in üblicher Weise die bekannten Säureadditionssalze herstellen. Alle Aussagen dieser Schrift und demgemäss der beanspruchte Schutzbereich beziehen sich daher sowohl auf die in freier Form vorliegenden Verbindungen als auch auf deren wasserlöslichen, physiologisch verträglichen Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Beispiele für die als Substituenten in den erfindungsgemäßen Verbindungen genannten C₁- bis C₄-Alkylgruppen sind die Gruppen Methyl, Ethyl, Propyl, Isopropyl und Butyl. Ethyl und Methyl sind bevorzugte Alkylgruppen. Bevorzugte C₁- bis C₂-Alkoxygruppen sind die Gruppen Methoxy und Ethoxy. Weiterhin können als bevorzugte Beispiele für eine C₁- bis C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Die übrigen verwendeten Bezeichnungen leiten sich von den hier gegebenen Definitionen ab.

Die m-Phenylendiaminderivate der Formel (I) lassen sich mit Hilfe herkömmlicher organischer Methoden herstellen. Beispielhaft sei an dieser Stelle auf die Versuchsdurchführungen im Rahmen der Ausführungsbeispiele verwiesen.

Einige der m-Phenylendiaminderivate der Formel (I) sind bereits aus dem Stande der Technik bekannt. Es ist der Literatur aber keinerlei Hinweis darauf zu entnehmen, dass diese als Kupplerkomponenten in Haarfärbemitteln eingesetzt werden können. Im Stand der Technik sind bisher nur sehr selten Oxidationsfarbstoffvorprodukte beschrieben, die eine Hydroxyalkylgruppe mit mehr als 4 Kohlenstoffatomen tragen. So werden beispielsweise in der WO-A-93/16 990 m-Phenylendiaminderivate offenbart, die mindestens einen ein Schwefelatom enthaltenden Substituenten aufweisen und deren Aminogruppen mit einer C₁-C₆-Monohydroxyalklygruppe substituiert sein können. Aber auch in dieser Schrift werden lediglich Derivate mit kurzen Alkylketten als konkrete Vertreter genannt. Dies spiegelt den Erfahrungswert wieder, dass größere Moleküle schlechter in das Haar eindringen können und somit üblicherweise zu Färbungen mit deutlich verschlechterten Echtheitseigenschaften führen. Es sind dem Stande der Technik somit keinerlei Hinweise zu entnehmen, dass die nunmehr beanspruchten m-Phenylendiaminderivate zu Färbungen führen, die die anwendungstechnischen Anforderungen im Hinblick auf die Echtheitseigenschaften erfüllen.

In Rahmen einer ersten Ausführungsform der vorliegenden Erfindung können die m-Phenylendiaminderivate bevorzugt sein, die mindestens ein asymmetrisches Zentrum im Substituenten X aufweisen. Die m-Phenylendiaminderivate der Formel (I), die unter diese Ausführungsform fallen, können als Gemisch der möglichen Stereoisomeren oder in der enantiomeren- beziehungsweise diastereomeren-reinen Form erfindungsgemäß eingesetzt werden. Falls im Rahmen dieser Anmeldung bei m-Phenylendiaminderivaten gemäß Formel (I) mit mindestens einem asymmetrischen Zentrum keine Angaben zur stereochemischen Konfiguration getroffen werden, sind unter diesen Verbindungen deren Racemate beziehungsweise Gemische der möglichen Stereoisomere zu verstehen. Der Einsatz der reinen Stereoisomere der entsprechenden m-Phenylendiaminderivate mit mindestens einem asymmetrischen Zentrum in den erfindungsgemäßen Mitteln soll ebenfalls umfasst sein.

Besonders bevorzugte Substituenten X im Rahmen dieser Ausführungsform sind ausgewählt aus der Gruppe, die gebildet wird von 1-Hydroxy-3,3-dimethyl-butan-2-yl, 1-Hydroxy-3-methyl-pentan-2-yl, 1-Hydroxy-pentan-2-yl, 1-Hydroxy-hexan-2-yl, 1-Hydroxy-4-methyl-pentan-2-yl, 6-Hydroxy-6-methyl-heptan-2-yl, 3-Hydroxy-2-methyl-hexan-2-yl und 5-Hydroxy-2-methyl-pentan-2-yl, (2R)-1-Hydroxy-3,3-dimethyl-butan-2-yl, (2S)-1-Hydroxy-3,3-dimethyl-butan-2-yl, (2R, 3R)-1-Hydroxy-3-methyl-pentan-2-yl, (2S, 3S)-1-Hydroxy-3-methyl-pentan-2-yl, 1-Hydroxy-3-methyl-butan-2-yl, (2S)-1-Hydroxy-3-methylbutan-2-yl, (2R)-1-Hydroxy-pentan-2-yl, (2S)-1-Hydroxy-pentan-2-yl, (2R)-1-Hydroxyhexan-2-yl, (2S)-1-Hydroxy-hexan-2-yl, (2R)-1-Hydroxy-4-methyl-pentan-2-yl, (2S)-1-Hydroxy-4-methyl-pentan-2-yl und (2S)-1-Hydroxy-4-methyl-pentan-2-yl.

Im Rahmen einer zweiten Ausführungsform der vorliegenden Erfindung können die m-Phenylendiaminderivate der Formel (I) bevorzugt sein, die kein asymmetrisches Zentrum im Substituenten X aufweisen. Im Rahmen dieser Ausführungsform kann es bevorzugt sein, wenn der Substituent X eine unverzweigten Monohydroxyalkylgruppe ist. Besonders bevorzugt sind die unverzweigten Monohydroxyalkylgruppen, die eine endständige Hydroxygruppe tragen.

Im Rahmen dieser Ausführungsform sind die Gruppen X bevorzugt, die ausgewählt sind aus 5-Hydroxypentyl, 6-Hydroxyhexyl, 7-Hydroxyheptyl, 4-Hydroxy-4-methyl-pentyl, 5-Hydroxy-4,4-dimethyl-pentyl und 3-Hydroxy-2,2-dimethyl-propyl.

Ganz besonders bevorzugte Gruppen X dieser Ausführungsform sind die 5-Hydroxypentylgruppe sowie die 6-Hydroxyhexylgruppe.

Erfindungsgemäß können die m-Phenylendiaminderivate der Formel (I) bevorzugt sein, bei denen das Stickstoffatom, das die Gruppe X trägt, weiterhin ein Wasserstoffatom trägt.

Weiterhin ist es erfindungsgemäß bevorzugt, wenn die Substituenten R¹, R², R³ und R⁴, die nicht für die Gruppen X stehen, für ein Wasserstoffatom, eine C₁- bis C₂-Alkylgruppe oder eine C₂-Monohydroxyalkylgruppe stehen. Besonders bevorzugt stehen diese Substituenten für ein Wasserstoffatom, eine Methylgruppe oder eine 2-Hydroxyethylgruppe. Ganz besonders bevorzugt sind m-Phenylendiaminderivate der Formel (I), bei denen die Substituenten R¹, R², R³ und R⁴, die nicht für die Gruppe X stehen, für ein Wasserstoffatom stehen.

Erfindungsgemäß können weiterhin die m-Phenylendiaminderivate der Formel (I) bevorzugt sein, bei denen R⁵ und R⁶ unabhängig voneinander für ein Wasserstoffatom, eine Methylgruppe, eine Methoxygruppe, eine Hydroxymethylgruppe oder eine 2-Hydroxyethylgruppe stehen.

Im Rahmen einer ersten Ausgestaltungsform der vorliegenden Erfindung können m-Phenylendiaminderivate der Formel (I) bevorzugt sein, bei denen mindestens einer der Reste R⁵ und R⁶ für ein Wasserstoffatom steht. Ganz besonders bevorzugt im Rahmen dieser Ausgestaltungsform sind die m-Phenylendiaminderivate der Formel (I), bei denen beide Reste R⁵ und R⁶ für ein Wasserstoffatom stehen.

Im Rahmen einer zweiten Ausgestaltungsform der vorliegenden Erfindung können m-Phenylendiaminderivate bevorzugt sein, bei denen mindestens einer der Reste R⁵ und R⁶ von einem Wasserstoffatom verschieden ist. Im Rahmen dieser Ausführungsform der vorliegenden Erfindung kann es besonders bevorzugte sein, dass einer der Reste R⁵ oder R⁶ für ein Wasserstoffatom steht und der andere der Reste R⁵ oder R⁶ für eine Methylgruppe steht. Es ist ganz besonders bevorzugt, wenn der von Wasserstoff verschiedene Rest sich in ortho-Stellung zu beiden Aminogruppen oder in para-Stellung zu einer der beiden Aminogruppe befindet. Obwohl erfindungsgemäß die m-Phenylendiaminderivate bevorzugt sind, bei denen nur einer der Reste R⁵ und R⁶ von einem Wasserstoffatom verschieden ist, steht prinzipiell nichts einem Einsatz der Derivate entgegen, bei denen sowohl R⁵ als auch R⁶ von einem Wasserstoffatom verschieden sind.

Erfindungsgemäß bevorzugte m-Phenylendiaminderivate der Formel (I) sind 1-(5-Hydroxypentyl)amino-3-aminobenzol, 1-(5-Hydroxypentyl)amino-2-methyl-3-aminobenzol, 1-(5-Hydroxypentyl)amino-4-methyl-3-aminobenzol, 1-(5-Hydroxypentyl)amino-5-methyl-3-aminobenzol, 1-(5-Hydroxypentyl)amino-2-methyl-5-aminobenzol, 1-(6-Hydroxyhexyl)amino-3-aminobenzol, 1-(6-Hydroxyhexyl)amino-2-methyl-3-aminobenzol, 1-(6-Hydroxyhexyl)amino-4-methyl-3-aminobenzol, 1-(6-Hydroxyhexyl)amino-5-methyl-3-aminobenzol und 1-(6-Hydroxyhexyl)amino-2-methyl-5-aminobenzol.

Erfindungsgemäß besonders bevorzugte m-Phenylendiaminderivate der Formel (I) sind 1-(5-Hydroxypentyl)amino-3-aminobenzol, 1-(5-Hydroxypentyl)amino-2-methyl-3-aminobenzol, 1-(5-Hydroxypentyl)amino-4-methyl-3-aminobenzol, 1-(6-Hydroxyhexyl)amino-3-aminobenzol, 1-(6-Hydroxyhexyl)amino-2-methyl-3-aminobenzol, und 1-(6-Hydroxyhexyl)amino-4-methyl-3-aminobenzo.

Ganz besonders bevorzugt im Rahmen der vorliegenden Erfindung sind N-(5-Hydroxypentyl)-m-phenylendiamin und N-(6-Hydroxyhexyl)-m-phenylendiamin.

Neben den m-Phenylendiaminderivaten der Formel (I) können die erfindungsgemäßen Färbemittel ferner mindestens eine Entwicklerkomponente enthalten.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 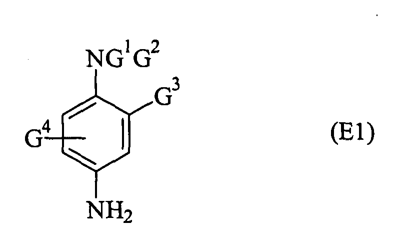 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 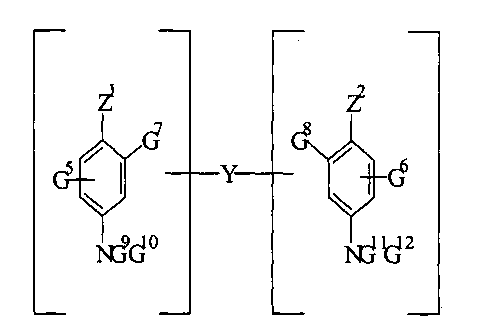 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 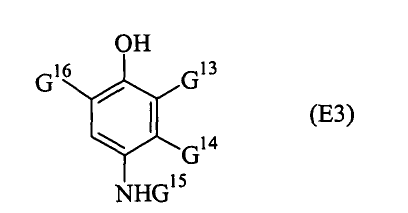 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁bis C₄)-Alkoxy-(C₁- bis C₄)-alkylrest, einen C₁- bis C₄-Aminoalkylrest, einen Hydroxy-(C₁bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methy(-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 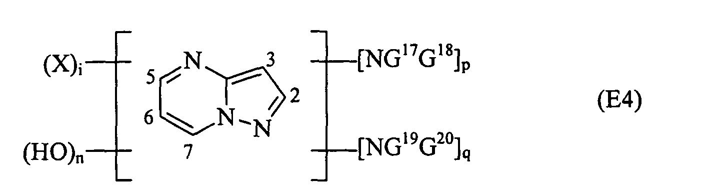 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

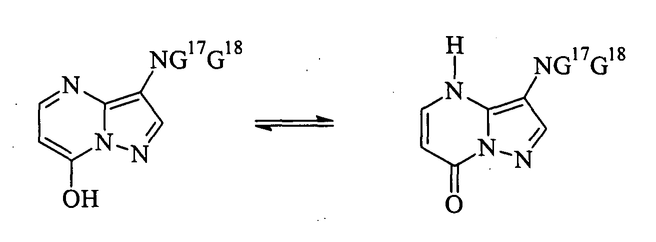

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine weitere Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als erfindungsgemäß besonders geeignet haben sich die folgenden Kuppler/Entwicklerkombinationen erwiesen:
- N-(5-Hydroxypentyl)-m-phenylendiamin / 4,5-Diamino-1-hydroxyethylpyrazol
- N-(5-Hydroxypentyl)-m-phenylendiamin / p-Toluylendiamin sowie
- N-(6-Hydroxyhexyl)-m-phenylendiamin / p-Toluylendiamin.

In einer weiteren Ausführungsform der vorliegenden Erfindung können die Färbemittel mindestens eine Vorstufe eines naturanalogen Farbstoffs enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIa),

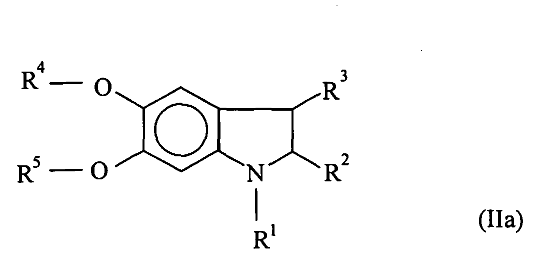

in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 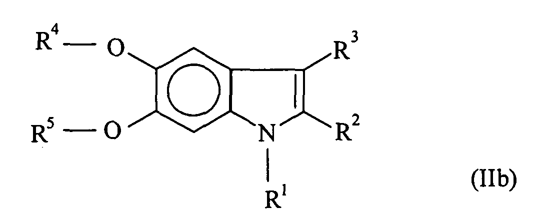 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den erfindungsgemäßen m-Phenylendiaminderivateb der Formel (I) können die erfindungsgemäßen Färbemittel in einer weiteren bevorzugten Ausführungsform der vorliegenden Erfindung zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

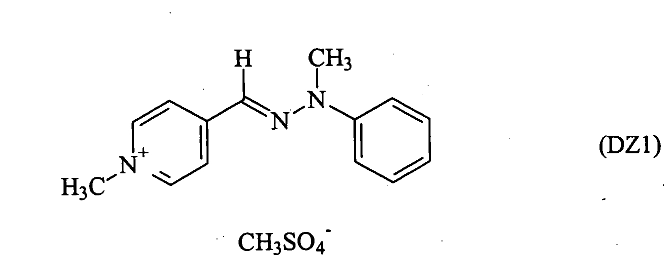

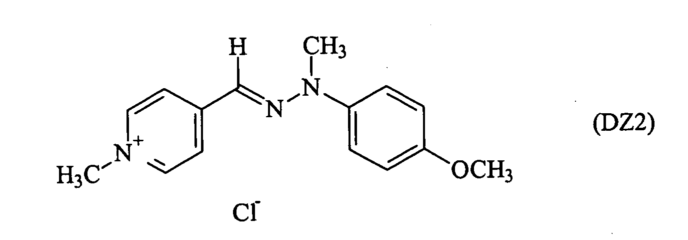

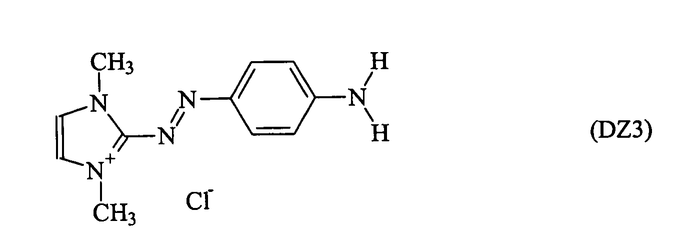

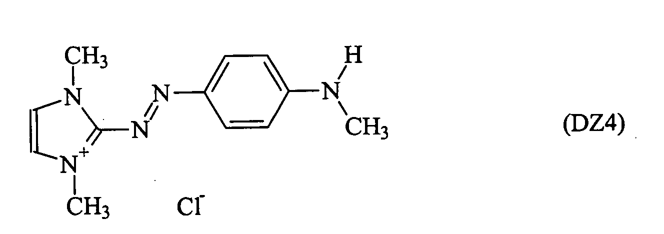

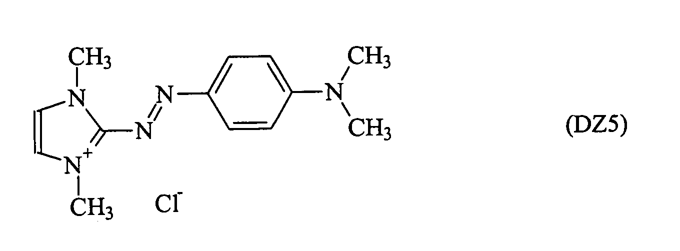

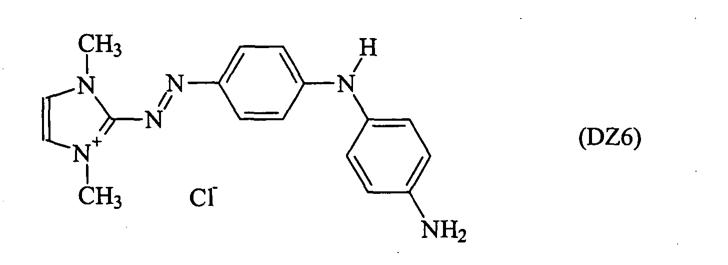

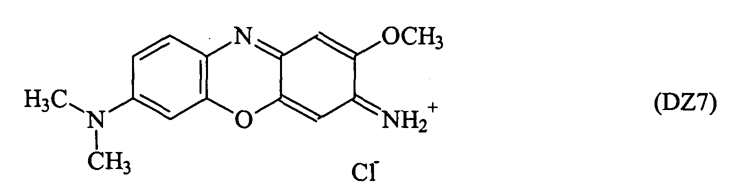

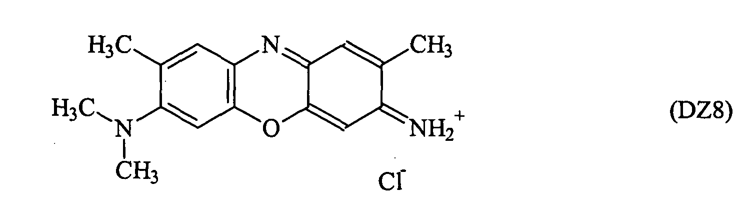

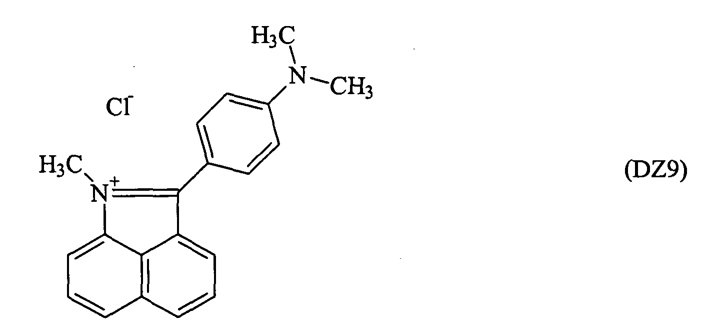

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykoloder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Anmeldung ist die Verwendung der erfindungsgemäßen m-Phenylendiaminderivate zum Färben keratinischer Fasern.

Ein dritter Gegenstand der vorliegenden Erfindung ist ein Verfahren zur Färbung keratinischer Fasern, bei dem ein erfindungsgemäßes Haarfärbemittel auf die Fasern aufgetragen wird und nach einer Einwirkzeit wieder abgespült wird.

Ein vierter Gegenstand der vorliegenden Erfindung ist N-(6-Hydroxyhexyl)-mphenylendiamin.

Ein fünfter Gegenstand der vorliegenden Erfindung sind m-Phenylendiaminderivate der Formel (II) 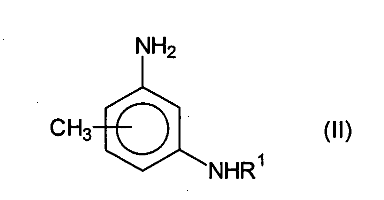 wobei
R¹steht für eine 5-Hydroxypentylgruppe oder eine 6-Hydroxyhexylgruppe.

Ein sechster Gegenstand der vorliegenden Erfindung sind 3-Nitroanilinderivate der Formel (III) 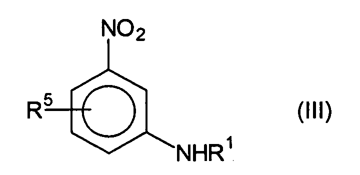 wobei
R¹steht für eine 5-Hydroxypentylgruppe oder eine 6-Hydroxyhexylgruppe und R⁵ steht für Wasserstoff oder eine Methylgruppe. Im Rahmen dieses Erfindungsgegenstandes sind die Zwischenstufen der Synthese der erfindungsgemäßen m-Phenylendiamine, ausgewählt aus der Gruppe, die gebildet wird von N-(5-Hydroxypentyl)-3-nitroanilin und N-(6-Hydroxyhexyl)-3-nitroanilin besonders bevorzugt.

### Ausführungsbeispiele

### 1 Synthesen

### 1.1 N-(5-Hydroxypentyl)-m-phenylendiamin

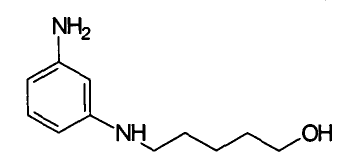

### 1.1.1 N-(5-Hydroxypentyl)-3-nitroanilin

28,2 g 3-Fluornitrobenzol und 113,4 g 5-Amino-1-pentanol wurden in 300 ml Dimethylsulfoxid gelöst, und der Ansatz wurde 60 h bei 100 °C gerührt. Nach dem Abkühlen wurde der Ansatz 1 I Wasser gegeben. Das Gemisch wurde mehrfach mit Methyl-*tert*.-butylether extrahiert und am Rotavapor bis zur Trockne eingeengt.

| | |
|---|---|
| Ausbeute | 40,5 g (90 %) |
| Schmelzpunkt | 71 - 75 °C |

### 1.1.2 N-(5-Hydroxypentyl)-m-phenylendiamin

40 g N-(5-Hydroxypentyl)-3-nitroanilin wurden in einer Mischung aus 450 ml Ethanol und 50 ml H₂O gelöst und mit 0,5 g Pd/C (5 %ig) versetzt. Nach Spülung mit Stickstoff wurde 12 h bei 50 °C mit 50 bar Wasserstoffdruck hydriert. Nach Abkühlung wurde erneut mit Stickstoff gespült, der Katalysator abfiltriert und das Filtrat am Rotavapor eingeengt. Es wurden 30 g schwarzes Öl erhalten, die im Kugelrohr destilliert wurden (120 - 204 °C, 0,06 - 0,08 mbar). Das Produkt wurde als oranges, dickflüssiges Öl erhalten.

| | |
|---|---|
| Ausbeute | 22,8 g (67 %) |

### 1.2 N-(6-Hydroxyhexyl)-m-phenylendiamin

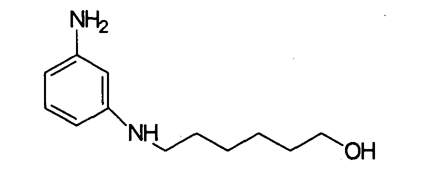

### 1.2.1 N-(6-Hydroxyhexyl)-3-nitroanilin

19,7 g 3-Fluornitrobenzol und 88,3 g 6-Amino-1-hexanol wurden in 300 ml Dimethylsulfoxid gelöst, und der Ansatz wurde 60 h bei 100 °C gerührt. Nach dem Abkühlen wurde der Ansatz in 1 I Wasser gegeben. Das Gemisch wurde mehrfach mit Methyl-*tert*.-butylether extrahiert und am Rotavapor bis zur Trockne eingeengt, wobei das Produkt als dunkelbraunes Öl erhalten wurde.

| | |
|---|---|
| Ausbeute | 29,0 g (87 %) |

### 1.2.2 N-(6-Hydroxyhexyl)-m-phenylendiamin

29 g N-(6-Hydroxyhexyl)-3-nitroanilin wurden in einer Mischung aus 450 ml Ethanol und 50 ml H₂O gelöst und mit 0,3 g Pd/C (5 %ig) versetzt. Nach Spülung mit Stickstoff wurde 12 h bei 50 °C mit 50 bar Wasserstoffdruck hydriert. Nach Abkühlung wurde erneut mit Stickstoff gespült, der Rückstand abfiltriert, und das Filtrat am Rotavapor eingeegnt. Es wurden 15,6 g schwarzes Öl erhalten, die im Kugelrohr destilliert wurden (120 - 201 °C, 0,0 - 0,1 mbar). Das Produkt wurde als hellbraune, kristalline Substanz erhalten.

| | |
|---|---|
| Ausbeute | 11,7 g (47 %) |
| Schmelzpunkt | 83 - 85 °C |

### 2 Ausfärbungen

### 2.1 Versuchsdurchführung

Für die Herstellung der Färbecreme wurden 50g einer Cremebasis in einem 250ml Becherglas eingewogen und bei 80°C geschmolzen. Die verwendete Cremebasis hatte die folgende Zusammensetzung:

| | |
|---|---|
| Hydrenol® D¹ | 17,0 Gew.-% |
| Lorol® tech.² | 4,0 Gew.-% |
| Texapon® NSO³ | 40,0 Gew.-% |
| Dehyton® K⁴ | 25,0 Gew.-% |
| Eumulgin® B2⁵ | 1,5 Gew.-% |
| Wasser | 12,5 Gew.-% |

| | |
|---|---|
| ¹ C₁₆₋₁₈-Fettalkohol (INCI-Bezeichnung: Cetearyl alcohol) (Cognis) | |
| ² C₁₂₋₁₈-Fettalkohol (INCI-Bezeichnung: Coconut alcohol) (Cognis) | |
| ³ Laurylethersulfat, Natriumsalz (ca. 27,5% Aktivsubstanz; INCI-Bezeichnung: Sodium Laureth Sulfate) (Cognis) | |
| ⁴ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) | |
| ⁵ Cetylstearylalkohol mit ca. 20 EO-Einheiten (INCI-Bezeichnung: Ceteareth-20) (Cognis) | |

Es wurden jeweils 1/400 Mol der Entwickler- bzw. Kupplerkomponente getrennt in destilliertem Wasser suspendiert bzw. unter Erwärmen gelöst. Anschließend wurde Ammoniak (<1 ml; 25%ige Ammoniaklösung) zugegeben bis der pH-Wert zwischen 9 und 10 lag.

Die gelösten Farbstoffvorprodukte wurden nacheinander in die heiße Creme eingearbeitet. Anschließend wurde mit destilliertem Wasser auf 97g aufgefüllt und mit Ammoniak ein pH-Wert von 9,5 eingestellt. Nach Auffüllen mit destilliertem Wasser auf 100 g wurde der Ansatz kaltgerührt (< 30°C), wobei eine homogene Creme entstand.

Für die Ausfärbungen wurden jeweils 25 g Färbecreme mit 25 g der folgenden Oxidationsmittelzubereitung vermischt.

| | |
|---|---|
| Dipicolinsäure | 0,1 Gew.-% |
| Natriumpyrophosphat | 0,03 Gew.-% |
| Turpinal® SL⁶ | 1,50 Gew.-% |
| Texapon® N28⁷ | 2,00 Gew.-% |
| Acrysol® 22⁸ | 0,60 Gew.-% |
| Wasserstoffperoxid, 50 %ig | 12,0 Gew.-% |
| Natronlauge, 45%ig | 0,80 Gew.-% |
| Wasser | ad 100 Gew.-% |

| | |
|---|---|
| ⁶ 1-Hydroxyethan-1,1-diphosphonsäure (ca. 58 - 61% Aktivsubstanzgehalt; INCI-Bezeichnung: Etidronic Acid, Aqua (Water)) (Solutia) | |
| ⁷ Laurylethersulfat-Natrium-Salz (mind. 26,5 % Aktivsubstanzgehalt; INCI-Bezeichnung: Sodium Laureth Sulfate) (Cognis) | |
| ⁸ Acrylpolymer (ca. 29.5 - 30.5% Festkörper in Wasser; INCI-Bezeichnung: Acrylates/Steareth-20 Methacrylate Copolyme) | |

In jede der so erhaltenen Mischungen wurde eine Haarsträhne (80 % ergraut; 330 mg bis 370 mg schwer) gegeben. Anschließend wurden die Mischungen und die Haarsträhnen auf jeweils ein Uhrglas gegeben und die Haarsträhnen in die Färbecremes gut eingebettet. Nach 30 Minuten (±1 Minute) Einwirkzeit bei Raumtemperatur wurden die Haarsträhnen entnommen und mit einer wässrigen Texapon® EVR-Lösung⁹ so oft gewaschen, bis der Farbüberschuß entfernt war. Die Haarsträhnen wurden an der Luft getrocknet und ihr Farbton unter der Tageslichtlampe (Farbprüfgerät HE240A) bestimmt und notiert (Taschenlexikon der Farben, A. Kornerup u. J.H. Wanscher, 3. unveränderte Auflage 1981, MUSTER-SCHMIDT Verlag; Zürich, Göttingen).
⁹ Laurylethersulfat-Natrium-Salz mit speziellen Zusätzen (ca. 34 bis 37% Aktivsubstanzgehalt; INCI-Bezeichnung: Sodium Lauryl Sulfate, Sodium Laureth Sulfate, Lauramide MIPA, Cocamide MEA, Glycol Stearate, Laureth-10) (Cognis)

Die bei den Ausfärbungs-Untersuchungen erhaltenen Ergebnisse sind in den nachstehenden Tabellen aufgeführt.

### 2.2 Ausfärbungen mit N-(5-Hydroxypentyl)-m-phenylendiamin

| | |
|---|---|
| Entwicklerkomponente | Ausfärbung |
| p-Toluylendiamin-sulfat | schwarzblau |
| 2,4,5,6-Tetraaminopyrimidin-sulfat | Oliv |
| p-Aminophenol | portweinrot |
| 4,5-Dimanio-1-(2-hydroxyethyl)pyrazol-sulfat | tiefmagenta |
| 1-(2-Hydroxyethyl)-2,5-p-phenylendiamin-sulfat | schwarzblau |
| 4-Amino-3-methylphenol | graubraun |
| Bis-(2-hydroxy-5-aminophenyl)-methan-dihydrochlorid | dunkelmagenta |

### 2.3 Ausfärbungen mit N-(6-Hydroxyhexyl)-m-phenylendiamin

| | |
|---|---|
| Entwicklerkomponente | Ausfärbung |
| p-Toluylendiamin-sulfat | schwarzblau |
| 2,4,5,6-Tetraaminopyrimidin-sulfat | Oliv |
| p-Aminophenol | violettbraun |
| 4,5-Dimanio-1-(2-hydroxyethyl)pyrazol-sulfat | dunkelmagenta |
| 1-(2-Hydroxyethyl)-2,5-p-phenylendiamin-sulfat | schwarzblau |
| 4-Amino-3-methylphenol | graubraun |
| Bis-(2-hydroxy-5-aminophenyl)-methan-dihydrochlorid | dunkelmagenta |

## Patentansprüche

1. Mittel zur Färbung keratinischer Fasern, insbesondere menschlicher Haare, enthaltend in einem kosmetisch akzeptablen Träger als Kupplerkomponente mindestens ein m-Phenylendiaminderivat der Formel (I) 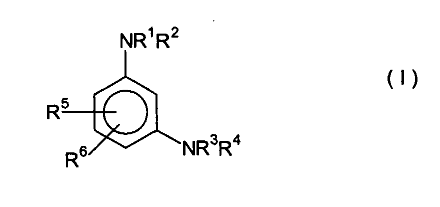 wobei
R¹, R², R³ und R⁴ stehen unabhängig voneinander für ein Wasserstoffatom, eine C₁- bis C₄-Alkylgruppe, eine C₂- bis C₄-Monohydroxyalkylgruppe oder R¹ und R² beziehungsweise R³ und R⁴ bilden gemeinsam mit dem sie tragenden Stickstoffatom einen Pyrrolidin-, einen Piperidin- oder Morpholinring, mit der Maßgabe, dass einer der Substituenten R¹, R², R³ oder R⁴ für eine lineare oder verzweigte, chirale oder achirale C₅- bis C₈-Monohydroxyalkylgruppe (X) steht, und R⁵ und R⁶ stehen unabhängig voneinander für ein Wasserstoffatom, eine C₁- bis C₂-Alkylgruppe, eine C₁- bis C₄-Monohydroxyalkylgruppe oder eine C₁- bis C₂-Alkoxygruppe.

2. Mittel nach Anspruch 1, **dadurch gekennzeichnet, dass** es mindestens ein m-Phenylendiaminderivat der Formel (I) enthält, das mindestens ein asymmetrisches Zentrum im Substituenten X aufweist.

3. Mittel nach Anspruch 2, **dadurch gekennzeichnet, dass** es ein m-Phenylendiaminderivat der Formel (I) enthält, bei dem der Substituent X ausgewählt ist aus der Gruppe, die gebildet wird von 1-Hydroxy-3,3-dimethylbutan-2-yl, 1-Hydroxy-3-methyl-pentan-2-yl, 1-Hydroxy-pentan-2-yl, 1-Hydroxyhexan-2-yl, 1-Hydroxy-4-methyl-pentan-2-yl, 6-Hydroxy-6-methyl-heptan-2-yl, 3-Hydroxy-2-methyl-hexan-2-yl und 5-Hydroxy-2-methyl-pentan-2-yl, (2R)-1-Hydroxy-3,3-dimethyl-butan-2-yl, (2S)-1-Hydroxy-3,3-dimethyl-butan-2-yl, (2R, 3R)-1-Hydroxy-3-methyl-pentan-2-yl, (2S, 3S)-1-Hydroxy-3-methyl-pentan-2-yl, 1-Hydroxy-3-methyl-butan-2-yl, (2S)-1-Hydroxy-3-methyl-butan-2-yl, (2R)-1-Hydroxypentan-2-yl, (2S)-1-Hydroxy-pentan-2-yl, (2R)-1-Hydroxy-hexan-2-yl, (2S)-1-Hydroxy-hexan-2-yl, (2R)-1-Hydroxy-4-methyl-pentan-2-yl, (2S)-1-Hydroxy-4-methyl-pentan-2-yl und (2S)-1-Hydroxy-4-methyl-pentan-2-yl.

4. Mittel nach einem der Ansprüche 1 bis 3, **dadurch gekennzeichnet, dass** es mindestens ein m-Phenylendiaminderivat der Formel (I) enthält, das kein asymmetrisches Zentrum im Substiuenten X aufweist.

5. Mittel nach Anspruch 4, **dadurch gekennzeichnet, dass** es ein m-Phenylendiaminderivat der Formel (I) enthält, bei dem die Gruppe X eine unverzweigten Monohydroxyalkylgruppe ist.

6. Mittel nach Anspruch 5, **dadurch gekennzeichnet, dass** es ein m-Phenylendiamin-Derivat der Formel (I) enthält, bei dem die Gruppe X eine unverzweigte Monohydroxyalkylgruppe mit einer endständigen Hydroxygruppe ist.

7. Mittel nach Anspruch 4, **dadurch gekennzeichnet, dass** es ein m-Phenylendiaminderivat der Formel (I) enthält, bei dem die Gruppe X ausgewählt ist aus 5-Hydroxypentyl, 6-Hydroxyhexyl, 7-Hydroxyheptyl, 4-Hydroxy-4-methylpentyl, 5-Hydroxy-4,4-dimethyl-pentyl und 3-Hydroxy-2,2-dimethyl-propyl.

8. Mittel nach einem der Ansprüche 1 bis 7, **dadurch gekennzeichnet, dass** es ein m-Phenylendiaminderivat der Formel (I) enthält, bei dem das Stickstoffatom, das die Gruppe X trägt, weiterhin ein Wasserstoffatom trägt.

9. Mittel nach einem der Ansprüche 1 bis 8, **dadurch gekennzeichnet, dass** es ein m-Phenylendiaminderivat der Formel (I) enthält, bei dem die Substituenten R¹, R², R³ und R⁴, die nicht für die Gruppe X stehen, für ein Wasserstoffatom stehen.

10. Mittel nach einem der Ansprüche 1 bis 9, **dadurch gekennzeichnet, dass** es ein m-Phenylendiaminderivat der Formel (I) enthält, bei dem mindestens einer der Reste R⁵ und R⁶ für ein Wasserstoffatom steht.

11. Mittel nach einem der Ansprüche 1 bis 9, **dadurch gekennzeichnet, dass** es ein m-Phenylendiaminderivat der Formel (I) enthält, bei dem mindestens einer der Reste R⁵ und R⁶ von einem Wasserstoffatom verschieden ist.

12. Mittel nach Anspruch 1 **dadurch gekennzeichnet, dass** das m-Phenylendiaminderivat der Formel (I) ausgewählt ist aus N-(5-Hydroxypentyl)-mphenylendiamin und N-(6-Hydroxyhexyl)-m-phenylendiamin.

13. Mittel nach einem der Ansprüche 1 bis 12, **dadurch gekennzeichnet, dass** es mindestens eine Entwicklerkomponente enthält.

14. Mittel nach einem der Ansprüche 1 bis 13, **dadurch gekennzeichnet, dass** es mindestens eine weitere Kupplerkomponente enthält.

15. Mittel nach einem der Ansprüche 1 bis 14, **dadurch gekennzeichnet, dass** es weiterhin mindestens einen direktziehenden Farbstoff enthält.

16. Mittel nach Anspruch 15, **dadurch gekennzeichnet, dass** der direktziehende Farbstoff kationisch ist.

17. Verfahren zur Färbung keratinischer Fasern, bei dem ein Mittel nach einem der Ansprüche 1 bis 16 auf die Fasern aufgetragen wird und nach einer Einwirkzeit wieder abgespült wird.

18. N-(6-Hydroxyhexyl)-m-phenylendiamin

19. m-Phenylendiaminderivat der Formel (II) 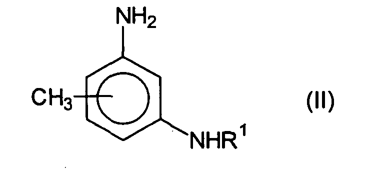 wobei
R¹steht für eine 5-Hydroxypentylgruppe oder eine 6-Hydroxyhexylgruppe.

20. 3-Nitroanilinderivat der Formel (III) 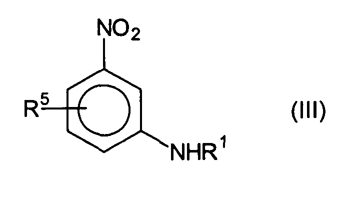 wobei
R¹steht für eine 5-Hydroxypentylgruppe oder eine 6-Hydroxyhexylgruppe und R⁵ steht für Wasserstoff oder eine Methylgruppe.
